# EUROPEAN PATENT APPLICATION

(11) **EP 4 166 527 A1**
(43) Date of publication of application: **19.04.2023**
(21) Application number: 21818172.5
(22) Date of filing: 28.05.2021
(51) Int. Cl.: C07C 1/12, C07C 9/04, F27D 17/00, C07B 61/00, C04B 7/44, C10L 3/08, B01D 53/14, B01D 53/62, C01B 32/50

(54) **METHOD FOR ACTIVATING CO2 IN EXHAUST GAS FROM CEMENT PRODUCTION, AND CO2 ACTIVATION SYSTEM**

(30) Priority: 04.06.2020 JP 2020097644; 04.06.2020 JP 2020097645
(71) Applicant: Mitsubishi Materials Corporation, Tokyo 100-8117 (JP)
(72) Inventor: TAKAYAMA, Yoshinori, Naka-shi, Ibaraki 311-0102 (JP); KAWASAKI, Hajime, Iwaki-shi, Fukushima 971-8101 (JP); KOMA, Takuma, Iwaki-shi, Fukushima 971-8101 (JP); KOMATSU, Takuya, Naka-shi, Ibaraki 311-0102 (JP)
(74) Representative: Gille Hrabal Partnerschaftsgesellschaft mbB Patentanwälte
(86) International application number: PCT/JP2021/020401
(87) International publication number: WO 2021/246313

(57) **Abstract**

Generating methane by adding hydrogen to CO₂ in exhaust gas discharged a from cement production facility or CO₂ that is separated and recovered from the exhaust gas, and using the methane as an alternative fuel to fossil fuel such as coal, petroleum, natural gas and the like, by methanation of CO₂ in the exhaust gas from the cement production facility that includes exhaust gas originated from lime stone not from the fossil oil and effectively utilizing it, it is possible to reduce usage of the fossil fuel, suppress CO₂ originated from energy, and improve an effect of reducing greenhouse gas.

## Description

### BACKGROUND OF THE INVENTION

### Technical Field

The present invention relates to a method of utilizing while reducing CO₂ originated from energy contained in an exhaust gas of cement production facility and a system of utilizing CO₂.

Priority is claimed on Japanese Patent Applications Nos. 2020-97644 and 2020-97645, filed Jun 4, 2020, the content of which is incorporated herein by reference.

### Background Art

In various combustion facilities such as thermal power generation or the like, in order to reduce greenhouse gas, it is made effort to reduce CO₂ that is generated and exhausted by combustion. Particularly, since most of energy which is necessary for a social activity is obtained by fossil fuel such as coal, petroleum, natural gas and the like and an amount of CO₂ generated from the foil fuel is stupendous, it is effective to reduce CO₂ originated from the energy for suppressing global warming.

As a technique for reducing CO₂ in combustion exhaust gas, conventionally, for example, the methanation method described in Patent Literature 1 is known. That is a method to obtain methane by separating carbon dioxide contained in the combustion exhaust gas to react with hydrogen. In this methanation method, a step of absorbing carbon dioxide in the combustion exhaust gas by bringing the combustion exhaust gas into contact with a carbon dioxide absorber, a step of taking out gas mainly having carbon dioxide by heating the carbon dioxide absorber that absorbed carbon dioxide, a step of removing sulfur compound in the gas by adding a first amount of hydrogen to the gas mainly having carbon dioxide and then throwing into a desulfurizer which is filled with desulfurizing agent, and a step of converting into methane by adding a second amount of hydrogen to the gas after the step of removing sulfur to convert by methanation reaction through methanation catalyst.

By using this methanation method, release of carbon dioxide from the combustion exhaust gas to the air is suppressed.

### Prior Art Document

### Patent Literature

Patent Literature 1: Japanese Unexamined Patent Application, First Publication No. 2019. 172595

### SUMMARY OF INVENTION

### Technical Problem

The method described in Patent Literature 1, the combustion gas which is exhausted can be effectively utilized by methanation. However, since when the methane produced from the combustion gas originated from fossil fuel is burned it is released as CO₂ again, it is impossible to say that the amount of CO₂ released from the fossil fuel is reduced.

The present invention is achieved in consideration of the above circumstances, and has an object to provide a method of utilizing CO₂ and a system of utilizing CO₂ in the exhaust gas of the cement production by methanation of CO₂ in the cement exhaust gas which is not originated from the fossil fuel to effectively utilize.

### Solution to Problem

A method for utilizing CO₂ in exhaust gas from cement production gas generates methane by adding hydrogen to CO₂ in exhaust gas from cement production facility or CO₂ separated and recovered from the exhaust gas and uses the methane as an alternative fuel to fossil fuel.

In the present invention, by converting CO₂ in the exhaust gas from the cement production facility or CO₂ separated and recovered from the exhaust gas into methane, CO₂ discharged from the cement production facility can be reduced and the methane can be effectively utilized by using this methane as the alternative fuel to the fossil fuel. The exhaust gas from the cement production facility contains a large amount of exhaust gas originated from lime stone, and the methane generated from the exhaust gas alternates the fossil fuel such as coal or petroleum, so that usage of the fossil fuel is reduced and CO₂ originated from energy can be decreased, and an effect of reducing greenhouse gas can be improved.

As alternatives to the fossil fuel, it can be fuel for cement burning kilns in the cement production facility, or can be used in various industrial facilities or the like such as a thermal power plant, an incineration facility of garbage or the like.

In this method for utilizing CO₂ in cement production exhaust gas, it is preferable that the CO₂ be CO₂ which is separated and recovered by bringing the exhaust gas from the cement production facility into contact with a CO₂ absorption material.

By using the CO₂ which is separated and recovered from the exhaust gas, concentration thereof is increased to produce methane having high concentration, so that the methane can be more effectively utilized. Amine or the like can be used for the CO₂ absorption material.

In this method for utilizing CO₂ in a cement production exhaust gas, the methane can be generated by directly adding the hydrogen to the exhaust gas.

A CO₂ utilizing system in cement production exhaust gas of the present invention is, in a cement production facility, provided with a methanation device that generates methane by adding hydrogen to CO₂ in exhaust gas from the cement production facility or CO₂ which is separated and recovered from the exhaust gas, and a methane supply device that supplies the methane as an alternative fuel to fossil fuel.

In this CO₂ utilizing system in cement production exhaust gas, it is preferable to have a CO₂ separation and recovery device that separates and recovers CO₂ by bringing the exhaust gas from the cement production facility into contact with a CO₂ absorption material to separate and recover CO₂.

In the CO₂ utilizing system in cement production exhaust gas, the methanation device may generate the methane by directly adding the hydrogen to the exhaust gas.

### Advantageous Effects of Invention

According to the present invention, since methane that is generated from the exhaust gas of the cement production facility is utilized as the alternative fuel to the fossil fuel, it is possible to utilize methane effectively, to reduce CO₂ originated from energy, and to improve the reduction effect of greenhouse gas.

### BRIEF DESCRIPTION OF DRAWINGS

[FIG. 1] It is a flowchart showing a process of a CO₂ utilizing method in cement production exhaust gas according to one embodiment of the present invention.
[FIG. 2] It is a view simply showing the CO₂ utilizing system in cement production exhaust gas of the above-described embodiment.
[FIG. 3] It is a block diagram showing a schematic composition of a methanation device configuring the CO₂ utilizing system in cement production exhaust gas of the above-described embodiment.

### DESCRIPTION OF EMBODIMENTS

An embodiment of a method for utilizing CO₂ in cement production exhaust gas and a CO₂ utilizing system in cement production exhaust gas of the present invention will be explained below referring drawings.

This embodiment is an example of generating methane from CO₂ in cement production exhaust gas and utilizing the methane as a part of alternative fuel to fossil fuel to a cement burning kiln and a calcination furnace.

### [Composition of CO₂ Utilizing System]

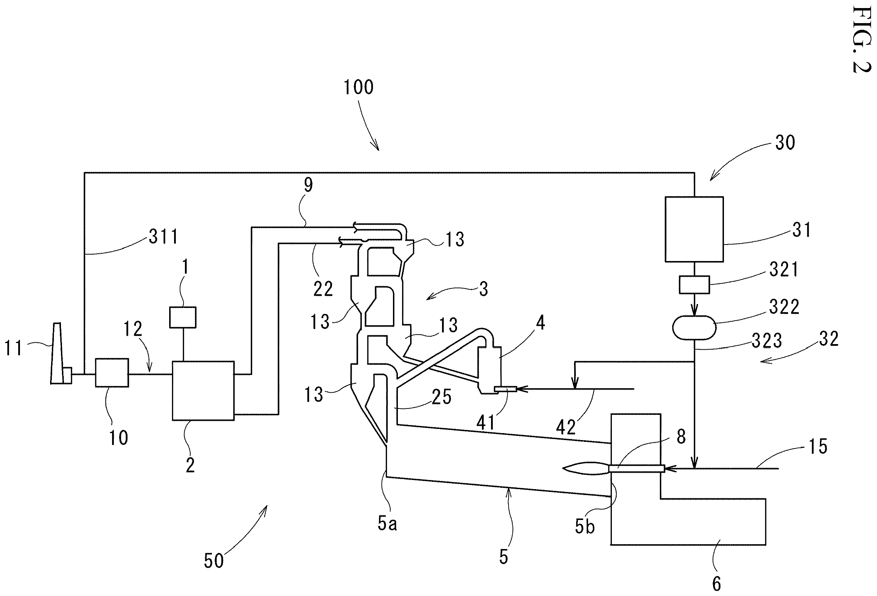

A CO₂ utilizing system 100 is provided with a cement production facility 50 and an exhaust gas treatment facility 30 used by connected to the cement production facility 50 as shown in FIG. 2. In this embodiment, the exhaust gas treatment facility 30 adds hydrogen to CO₂ to the exhaust gas from the cement production facility 50 or CO₂ that is separated and recovered from the exhaust gas to generate methane, and supplies the generated methane as an alternative fuel to a part or entire of the fossil fuel to the cement production facility 50.

### [Composition of Cement Production Facility]

The cement production facility 50 is provided with, as the whole is shown in FIG. 2, a raw material storehouse 1 individually storing lime stone, clay, silica stone, iron material and the like as cement materials, a material mill and a dryer (hereinafter "material mill/dryer") 2 milling and drying these cement materials, a preheater 3 preheating the powdery cement materials supplied via a material supply pipe 22 and obtained by the material mill, a calcination furnace 4 calcining the cement materials preheated by the preheater 3, a cement burning kiln 5 burning the cement materials that are calcined, and a cooler 6 and the like to cool cement clinker after burned in the cement burning kiln 5.

The cement burning kiln 5 is a lateral cylindrical rotary kiln which is slightly inclined, rotated around an axis to send the cement materials supplied in a kiln tail part 5a from the preheater 3 to a kiln front part 5b, and heats and burns to generate cement clinker at about 1450°C by a burner 8 at the kiln front part 5b while sending. The generated cement clinker is sent out from the kiln front part 5b to the cooler 6. To the burner 8, a fuel supply line 15 supplying fuel containing fossil fuel such as coal, petroleum and the like is connected. Other than the fuel supply line 15, in order to enlarge the heat energy, a supplying system (not illustrated) of an alternative heat source such as waste plastic, waste tires and the like is also provided. The cement clinker is cooled in the cooler 6 to a prescribed temperature, then sent to a finishing step.

The preheater 3 is configured so that a plurality (four in an example shown in FIG. 2) of cyclones 13 that flow the exhaust gas occurs in the cement burning kiln 5 are vertically connected as shown in FIG. 2. Between the lowermost cyclone 13 and the next cyclone 13, the calcination furnace 4 is connected. The cement material calcined by the combustion gas of the calcination furnace 4 is supplied to the kiln tail part 5a of the cement burning kiln 5 from the lowermost cyclone 13.

The calcination furnace 4 has a burner 41 therein, and burns fuel such as coal or the like supplied from a fuel supply line 42, thereby calcining the cement material sent from the upper cyclone 13, and supplying the calcined cement material to the lowermost cyclone 13 through a riser duct 25 together with the exhaust gas generated by the calcination. The cement material is supplied from the lowermost cyclone 13 to the kiln tail part 5a of the cement burning kiln 5. The riser duct 25 sends the exhaust gas from the kiln tail part 5a of the cement burning kiln 5 to the lowermost cyclone 13, and the exhaust gas occurs in the calcination furnace 4 is also supplied to the cyclone 13 through the riser duct 25. Therefore, the exhaust gas of the cement burning kiln 5 and the exhaust gas from the calcination furnace 4 go together thorough the preheater 3 from the lower side to the upper side and then are introduced into the material mill/dryer 2 passing through an exhaust pipe 9.

The material mill/dryer 2 carries out pulverization and drying of the cement material simultaneously by introducing the exhaust gas from the calcination furnace 4 and the cement burning kiln 5. To the material mill/dryer 2, an exhaust gas treatment line 12 having a dust collector 10, a chimney 11 and the like is connected.

### [Configuration of Exhaust Gas Treatment Facility]

The exhaust gas treatment facility 30 is provided with an exhaust gas collection line 311 collecting the exhaust gas occurred in the cement burning kiln 5 and the calcination furnace 4 before discharged from the chimney 11 a methanation device 31 separating and recovering CO₂ from the exhaust gas sent from the exhaust gas collection line 311 and adding hydrogen to the separated and recovered CO₂ to generate methane, and a methane supply device 32 supplying the generated methane to the cement production facility 50.

The exhaust gas collection line 311 is connected between the dust collector 10 and the chimney 11 in the exhaust gas treatment line 12 of the cement production facility 50, and collects a part of the exhaust gas generated during cement burning. Since it is the exhaust gas is generated by burning of cement, an exhaust gas due to combustion of fuel such as coal is partially included, but it contains a large amount of exhaust gas originated from lime stone.

### (Configuration of Methanation Device)

The methanation device 31 is provided with a CO₂ separation/recover device 310 that separates and recovers CO₂ from the exhaust gas, a hydrogen mixing unit 316 that supplies and mixes hydrogen (for example, hydrogen gas) to CO₂ separated and recovered by the CO₂ separation/recover device 310 and a methane production part 317 that generates methane from CO₂ in which hydrogen is mixed.

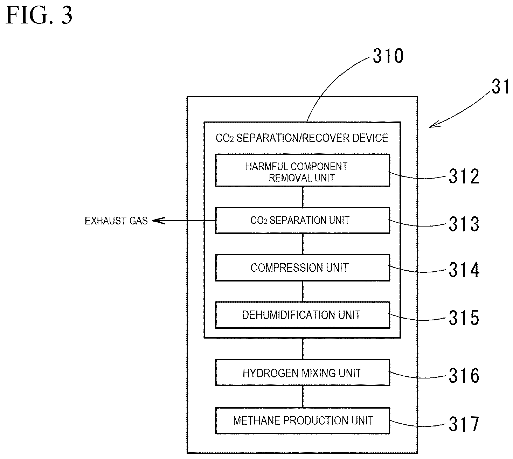

As shown in FIG. 3, the CO₂ separation/recover device 310 is provided with, a harmful component removal unit 312 that removes harmful components such as SOₓ, NOₓ and the like from the exhaust gas collected in the exhaust gas collection line 311, a CO₂ separation/recover unit 313 that separates and recovers CO₂ from the exhaust gas from which the harmful components are removed, a compression unit 314 that compresses the recovered CO₂, and a dehumidification unit 315 that removes moisture from the compressed CO₂.

The exhaust gas sent from the exhaust gas collection line 311 may contain not only combustion exhaust gas of fossil fuel such as coal, petroleum coke, and heavy oil but also combustion exhaust gas of waste plastic or waste tire. In that case, CO₂ is contained with about 20% for example, and a gas other than CO₂ and a harmful component are included. Therefore, the harmful component removal unit 312 removes the harmful component (for example, acidification gas such as nitric oxide [NOₓ] or sulfur oxide [SOₓ]) from the exhaust gas, and is provided with a scrubber that is filled with an aqueous NaOH and the like. By removing the harmful component, since halogen is also removed with NOₓ, an absorber of amine compound used for the next separation/recover of CO₂ is prevented from deteriorating.

The CO₂ separation/recover unit 313 is made of a standard CO₂ recover device, and is provided with a CO₂ absorbent material (a liquid absorbent material in which an amine compound is dissolved in water, a solid absorbent material in which an amine compound is supported on a porous material, and the like) that absorbs CO₂ in the exhaust gas when the exhaust gas in which the harmful components are removed comes into contact therewith. Then, by heating the CO₂ absorbent material that has absorbed CO₂ and the like, CO₂ is taken out from the CO₂ absorbent material and recovered. The CO₂ separation/recover unit 313 discharges the exhaust gas after removing CO₂ to the exterior. The compression unit 314 compresses the recovered CO₂ by applying a pressure of 0.1 MPa or more, preferably 0.5 to 1.0 MPa. The dehumidification unit 315 removes moisture contained in CO₂ by cooling the compressed CO₂. This dehumidification is carried out in order to remove moisture before methanation since the moisture affects the oxidation of a Ni-based catalyst in the methanation device.

The hydrogen mixing unit 316 supplies hydrogen to the dehumidified CO₂, mixes, and compresses it. The hydrogen produced by artificial light synthesis using renewable energy, decomposition of water, or the like can be utilized. The amount of hydrogen added by the hydrogen mixing unit 316 is appropriately set so that methane can be easily produced from CO₂ in which hydrogen is mixed.

The methane production unit 317 generates methane from CO₂ in which hydrogen is mixed. The methane production unit 317 is composed of a general methane production apparatus, provided with a plurality of reactors (not illustrated) filled with a catalyst exhibiting activity in methanation, and produces methane by supplying and reacting CO₂ in which hydrogen is mixed with these reactors. For example, Ni, Pt, Pd, and Cu are used as a hydrogenation catalyst; and as the methanation catalyst, particularly, Ni and Ni alloy on which Al₂O₃, Cr₂O₃, SiO₂, MgAl₂O₄, TiO₂, ZrO₂ or the like are supported.

### (Configuration of Methane Supply Device)

As shown in FIG. 2, the methane supply device 32 is provided with a tank 322 compressing methane produced by the methanation device 31 by a pump 321 and storing it, and a methane supply line 323 that is connected to the tank 322 and sends methane to the burner 8 of the kiln front part 5b and the burner 41 of the calcination furnace 4 respectively. The methane supply line 323 is connected to the fuel supply line 15 supplying fuel such as coal, petroleum or the like to the burner 8 of the cement burning kiln 5, and the fuel supply line 42 supplying fuel such as coal or the like to the burner 41 of the calcination furnace 4. As a result, methane is supplied to the burners 8 and 41 together with the fuel.

### [CO₂ Utilizing Method]

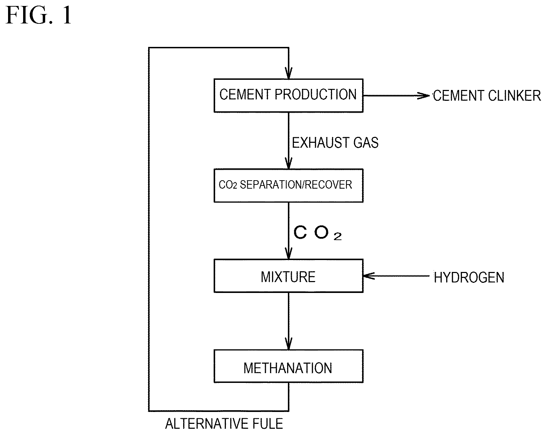

A method of reducing CO₂ in the exhaust gas of the cement production facility 50 and effectively utilizing it using the above-described CO₂ utilizing system will be explained with the flowchart shown in FIG. 1.

In the cement production facility 50, the powdery cement material obtained by milling and drying lime stone, clay, silica stone, iron material and the like as the cement material is preheated; the preheated cement material is subjected to the calcination and then burned, and cooled, so that the cement clinker is produced. The exhaust gas occurred in the cement burning kiln 5 and the calcination furnace 4 during the production of the cement clinker goes through the preheater 3 from the lower side to the upper side, passes through the exhaust pipe 9 to be introduced into the material mill/drier 2 for drying the cement material, then is discharged from the chimney 11 via the dust collector 10.

In this process of producing cement, a part of the exhaust gas occurred when the cement is burned is collected to the exhaust gas collection line 311 of the methanation device 31 between the dust collector 10 and the chimney 11 of the exhaust gas treatment line 12. Next, the harmful component removal unit 312 removes the harmful component from the exhaust gas. In the harmful component removal unit 312, nitrogen oxide (NOx), sulfur oxide (SOx), halogen and the like are removed. Then, CO₂ is taken out from the exhaust gas by the CO₂ separation/recover unit 313 and separated/recovered. At this time, the exhaust gas from which CO₂ is removed is discharged outside.

Next, the compression unit 314 compresses the recovered CO₂ to be 0.1 MPa or more by applying a pressure of 0.5 to 1.0 MPa, and then moisture included in CO₂ is removed by the dehumidification unit 315. Then, by the hydrogen mixing unit 316, hydrogen is supplied to the dehumidified CO₂ and mixed with it, then pressurized. Then, by the methane production unit 317, methane is generated from CO₂ in which hydrogen is mixed.

The methane generated in this manner is stored in the tank 322 of the methane supply device 32. The methane stored in the tank 322 is supplied to the cement burning kiln 5 and the calcination furnace 4 via the methane supply line 323. In the cement burning kiln 5, fossil fuel such as petroleum, coal of the like is supplied from the fuel supply line 15, however, some of the fossil fuel can be substituted with methane by supplying methane, and the fossil fuel can be reduced. Similarly, in the calcination furnace 4, some of the fuel such as coal is substituted with methane, so that fossil fuel can be reduced.

In the present embodiment, CO₂ separated and recovered from the exhaust gas from the cement production facility 50 is converted into methane, so that CO₂ discharged from the cement production facility 50 can be reduced, and the methane can be effectively utilized by using the methane as an alternative fuel for the cement burning kiln 5 and the calcination furnace 4. In particular, since the fossil fuel such as coal or petroleum that is a major cause of global warming is substituted with methane derived from limestone, it is possible to reduce the use of fossil fuel, reduce CO₂ of energy source, and increase the effect of reducing greenhouse gas. Moreover, using CO₂ separated and recovered from the exhaust gas, high concentration methane can be produced, and methane can be more effectively used.

The present invention is not limited to the above-described embodiments and various modifications may be made without departing from the scope of the present invention.

For example, in the above embodiment, CO₂ is separated and recovered from the exhaust gas of the cement production facility 50, however, since CO₂ is contained in the exhaust gas of the cement production facility 50 at a concentration of 20% and more, methane may be generated by adding hydrogen directly to the exhaust gas.

In addition, although the generated methane is supplied to both the cement burning kiln 5 and the calcination furnace 4, it may be supplied to either one of them.

Furthermore, although the methane is generated using the exhaust gas of both the cement burning kiln 5 and the calcination furnace 4, it is also possible to apply to a cement production facility having no calcination furnace; in that case, methane is generated from the exhaust gas from a cement burning kiln.

Moreover, in the present embodiment, although the methane generated from the exhaust gas from the cement production facility 50 is supplied to the cement burning kiln 5 and the calcination furnace 4 of the cement production facility 50 so it is utilized as the alternative fuel to the fossil fuel used in the cement production facility 50, but the present invention is no limited to this, it may be used as an alternative fuel to fossil fuel on the other various apparatuses and facilities. For example, it is used in a wide variety of applications as alternative fuel of fossil fuels such as coal, petroleum, LNG and the like used in thermal power plants, petroleum refining facilities, natural gas purification facilities, and waste incineration facilities of waste and the like, fuel cells, and various industrial facilities, and as an alternative fuel used in ordinary homes to alternate city gas.

### Industrial Applicability

It is possible to effectively use methane generated from an exhaust gas of a cement production facility as an alternative fuel to fossil fuel, reduce CO₂ derived from energy, and increase an effect of reducing greenhouse gas.

### Reference Signs List

- 1: Raw material store house
- 2: material mill/dryer
- 3: Preheater
- 4: Calcination furnace
- 5: Cement burning kiln
- 5a: Kiln tail part
- 5b: Kiln front part
- 6: Cooler
- 8: Burner
- 9: Exhaust pipe
- 10: Dust collector
- 11: Chimney
- 12: Exhaust gas treatment line
- 13: Cyclone
- 15: Fuel supply line
- 22: Material supply pipe
- 25: Riser duct
- 30: Exhaust gas treatment facility
- 31: Methanation device
- 310: CO₂ separation/recover device
- 311: Exhaust gas collection line
- 312: Harmful component removal device
- 313: CO₂ separation/recover unit
- 314: Compression unit
- 315: Dehumidification unit
- 316: Hydrogen mixing unit
- 317: Methane production unit
- 32: Methane supply device
- 321: Pump
- 322: Tank
- 323: Methane supply line
- 41: Burner
- 42: Fuel supply line
- 50: Cement production facility
- 100: CO₂ utilizing system

## Claims

1. A method for utilizing CO₂ in cement production exhaust gas, wherein methane is generated by adding hydrogen to CO₂ in the exhaust gas from cement production facility or CO₂ which is separated and recovered from the exhaust gas, to use the methane as an alternative fuel to fossil fuel.

2. The method for utilizing CO₂ in cement production exhaust gas according to claim 1, wherein the CO₂ is CO₂ which is separated and recovered by bringing the exhaust gas from the cement production facility into contact with a CO₂ absorption material.

3. The method for utilizing CO₂ in cement production exhaust gas according to claim 1, wherein the hydrogen is directly added to the exhaust gas to generate methane.

4. A CO₂ utilizing system in cement production exhaust gas, comprising in a cement production facility, a methanation device that generates methane by adding hydrogen to CO₂ in exhaust gas from the cement production facility or CO₂ which is separated and recovered from the exhaust gas, and a methane supply device that supplies the methane as an alternative fuel to fossil fuel.

5. The CO₂ utilizing system in cement production exhaust gas according to claim 4, comprising a CO₂ separation and recovery device that separates and recovers CO₂ by bringing the exhaust gas from the cement production facility into contact with a CO₂ absorption material to separate and recover CO₂.

6. The CO₂ utilizing system in cement production exhaust gas according to claim 4, wherein the methanation device generates the methane by directly adding the hydrogen to the exhaust gas.
